# EUROPÄISCHE PATENTANMELDUNG

(11) **EP 1 099 412 A2**
(43) Veröffentlichungstag der Anmeldung: **16.05.2001**
(21) Anmeldenummer: 00123646.2
(22) Anmeldetag: 30.10.2000
(51) Int. Cl.: A61B 17/16, A61C 1/00

(54) **Highspeedmotor für die chirurgische Bearbeitung von Knochen**

(30) Priorität: 13.11.1999 DE 19954717
(71) Anmelder: Von Zeppelin, Dieter, 82049 Pullach (DE)
(72) Erfinder: Von Zeppelin, Dieter, 82049 Pullach (DE)
(74) Vertreter: Becker Kurig Straus

(57) **Zusammenfassung**

Die Erfindung betrifft einen Highspeedmotor für die chirurgische Knochenbearbeitung und Mikrotrepanation, mit einem Motorgehäuse 1, 1a und einem Aufnahmefutter 7 für einen Fräserstift 8, wobei das Aufnahmefutter 7 einen lösbaren Verriegelungsmechanismus 9 aufweist. Das Motorgehäuse ist (1, la) einstückig und abgewinkelt ausgeführt und weist in seinem vorderen Bereich (1a) ein Auflageteil (4) für die Fingerablage des Benutzers auf.

## Beschreibung

Die vorliegende Erfindung betrifft einen mit Luftdruck, Stickstoff, Nitrogen o.ä. betriebenen Highspeedmotor für die chirurgische Bearbeitung von Knochen und für die Mikrotrepanation, insbesondere bei spinalen und kraniellen Eingriffen in der Neurochirurgie, Orthopädie sowie im HNO-Bereich.

Mit Highspeedmotoren wird bei einer Umdrehungszahl des Motors von über 70.000 Upm der Knochen präzise, schnell und ohne nennenswerten Kraftaufwand des Chirurgen abgetragen. Solche Systeme haben sich im Bereich der Mikrotrepanation in den letzten zehn Jahren verstärkt durchgesetzt. Die operative Sicherheit wird wesentlich erhöht.

Es sind bereits ähnliche Systeme bekannt, die durch Luftdruck, Stickstoff, Nitrogen, Strom o.ä. betrieben werden und zu einem ähnlichen Zweck eingesetzt werden.

Aus der DE 41 03 663 C2 ist ein chirurgisches Handstück bekannt, das eine geradlinige, lineare Form aufweist.

Aus der DE-OS 27 22 334 ist ein chirurgisches Instrument, insbesondere zur Ohrenchirurgie, bekannt, das eine Abwinkelung des Gehäuses zeigt.

Aus der DE 34 07 199 A1 ist ein Schneidhandstück mit einer Kühlmittelvorrichtung bekannt, das zur Zahnbehandlung eingesetzt wird.

Aus dem deutschen Gebrauchsmuster 7509547.5 ist ein pneumatisch angetriebener chirurgisches Drehgerät bekannt, das eine lineare, geradlinige Struktur im Gehäusebereich aufweist.

Aus dem deutschen Gebrauchsmuster 7536182 ist eine Einrichtung zur Drehmomentübertragung bekannt, die ebenfalls eine längliche, geradlinige Gehäusestruktur hat.

Aus dem deutschen Gebrauchsmuster 7332292.6 ist ein chirurgisches Instrument bekannt, das ein linear langgestrecktes Gehäuse aufweist.

Bei den vorgenannten Motordrillsystemen ist jedoch die Ergonomie bei der Handhabung nicht ausreichend bedacht worden. Die genannten Motordrillsysteme sind zum Teil unhandlich, in der Bedienung aufwendig und stellen den Chirurgen vor Handhabungsprobleme.

Zusätzlich ist zu beanstanden, dass die aufwendige Konstruktion der bekannten Motordrillsysteme einen hohen Herstellungspreis zur Folge hat.

Aufgabe der vorliegenden Erfindung ist die Angabe eines Highspeedmotors für die chirurgische Knochenbearbeitung, der ergonomisch gestaltet ist und eine entspannte, ermüdungsfreie und präzise Führung des Arbeitsinstruments ermöglicht. Dies wird mit einem Highspeedmotor gemäß Anspruch 1 erreicht. Die abgewinkelte Ausbildung des Motorgehäuses ermöglicht eine sichere Positionierung des Highspeedmotors in der linken wie in der rechten Hand des Benutzers. Damit liegt der Highspeedmotor gemäß der Erfindung griffig in der Hand des Benutzers und ist lagestabil. Aufgrund der einstückigen Ausbildung des Motorgehäuses ist eine Gewichtsersparnis zu verzeichnen, die auch zu geringeren Herstellungskosten führt. An die Stelle mehrerer miteinander zu verbindender Gehäuseteile tritt ein einziger einstückiger Gehäuseteil.

Das Auflageteil weist bevorzugt zwei einander gegenüberliegende Auflageflächen für die Daumen- bzw. Fingerablage auf. Dadurch werden für die das Gehäuse haltenden Finger Ablageflächen geschaffen, die das Motorgehäuse zusätzlich lagestabilisieren.

Die Auflageflächen sind bevorzugt gegeneinander abgeschrägt bzw. laufen aufeinander zu, so dass zwischen drei anliegenden Fingern des Benutzers eine sichere und präzise Führung des Motors wird.

Das Auflageteil ist bevorzugt verschieblich gelagert und mit dem Verriegelungsmechanismus verbunden. Dadurch wird das Auflageteil mit einer weiteren Funktion ausgestattet und ist geeignet, den eingesteckten Fräserstift zu ver- und entriegeln.

Ein Kupplungsstück ist bevorzugt mit dem hinteren Bereich des Motorgehäuses verbunden, wobei das Kupplungsstück für den Anschluß einer Antriebsmittel-Zuleitung dient. Hierunter wird vorzugsweise ein Druckluftanschluß o.ä. verstanden.

Zur Führung und Abdeckung des Fräserstifts ist bevorzugt ein Schaftaufsatz vorgesehen, der eine Hüllmutter aufweist, wobei die Hüllmutter am Motorgehäuse befestigbar ist. Damit ist der Fräserstift wirksam geführt und geschützt.

Das Auflageteil liegt bevorzugt an der Hüllmutter ohne wesentlichen Spalt an, wenn der Fräserstift verriegelt ist. Dadurch läßt sich der Verriegelungszustand des Fräserstifts optisch kontrollieren.

Die Winkelung des Gehäuses des Highspeedmotors beträgt zwischen 20° und 45°. In diesem Bereich der Abwicklung hat sich eine gute Ergonomie in der Praxis herausgestellt .

Bevorzugt dient eine Ablagefläche des Auflageteils als Mittelfingerauflage und eine andere Ablagefläche als Daumenauflage.

Bevorzugt ist das Auflageteil bzw. seine Ablagefläche profiliert.

Bevorzugt ist die Hüllmutter zur Auflage für die Fingerspitzen des Benutzers ausgebildet und ist bevorzugt zur Erhöhung der Griffigkeit mit einem Rillenprofil oder dgl. ausgestattet.

Das Kupplungsstück des Highspeedmotors ist bevorzugt mit einem doppellumigen bzw. mehrlumigen Druckluftschlauch oder alternativ mit einem Winkeladapter verbindbar.

Der Winkeladapter ist bevorzugt mit einem doppellumigen bzw. mehrlumigen Druckluftschlauch verbindbar.

Die Verbindungen von Kupplungsstück und Winkeladapter bzw. Druckluftschlauch erfolgen bevorzugt mittels Stift- oder Bajonettverschluß.

Es ist bevorzugt, dass der vordere Gehäuseteil gegenüber dem hinteren Gehäuseteil im Querschnitt verkleinert ist. Dies trägt der Tatsache Rechnung, dass durch den Ansatz für einen Schaftaufsatz nach der Abwinkelung das Aufnahmefutter wesentlich verschlankt werden kann. Ein weiterer Vorteil ist die Gewichtsersparnis durch den kleineren Durchmesser und die Kostenersparnis durch den Wegfall von gewinkelten Schaftaufsätzen.

Weitere Vorteile, Merkmale und Anwendungsmöglichkeiten der vorliegenden Erfindung ergeben sich aus der nachfolgenden Beschreibung eines Ausführungsbeispiels in Verbindung mit der Zeichnung.
- Fig. 1: ist eine Seitenansicht einer Ausführungsform eines erfindungsgemäßen Highspeedmotors mit gerader Schlauchbefestigung;
- Fig. 2: ist eine Seitenansicht einer weiteren Ausführungsform eines erfindungsgemäßen Highspeedmotors mit Winkeladapter und daran anschließender Schlauchbefestigung;
- Fig. 3: ist eine Seitenansicht eines Winkeladapters zur Verwendung in einer Ausführungsform der Erfindung;
- Fig. 4: ist eine Seitenansicht und eine Aufsicht eines Auflageteils zur Verwendung in einem erfindungsgemäßen Highspeedmotor;
- Fig. 5: ist ein Querschnitt durch die Ausführungsform von Fig. 2.

Der in den Figuren dargestellte Highspeedmotor weist ein einstückiges Gehäuse auf, das die miteinander winklig verbundenen Gehäusebereiche 1a, 1 enthält. Dabei ist der vordere Gehäusebereich la im Querschnitt gegenüber dem hinteren Gehäusebereich 1 verjüngt bzw. verkleinert. Das Motorgehäuse 1, 1a ist mit einer Winkelung von etwa 30° ausgelegt und damit geeignet, gut balanciert in der Hand eines Benutzers zu liegen. Dies gilt für die rechte und für die linke Hand eines Benutzers.

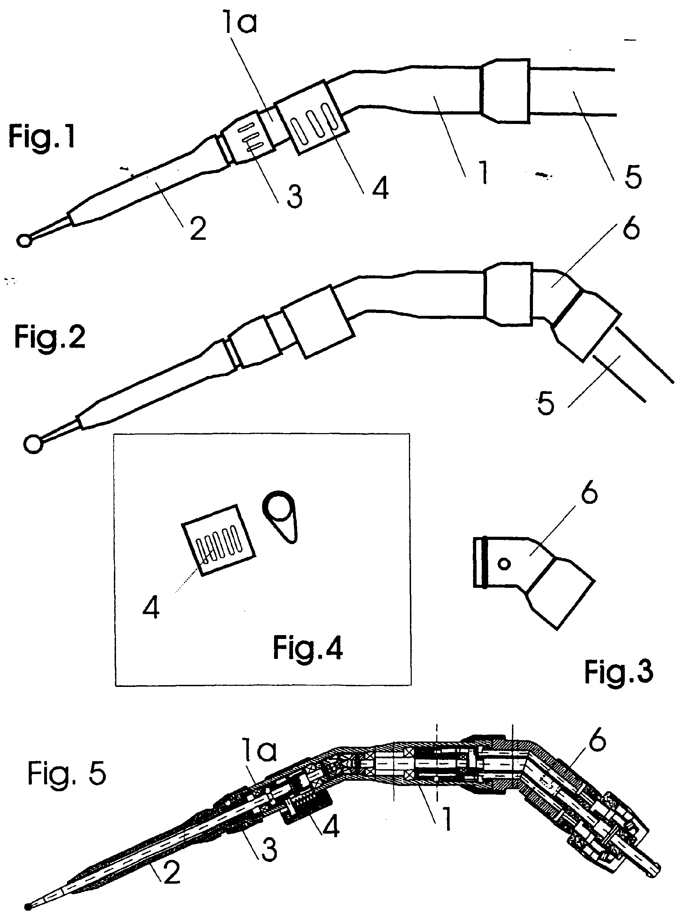

Im vorderen Gehäuseteil 1a ist ein Auflageteil 4 für die Fingerablage des Benutzers gebildet, das in größerem Detail in Fig. 4 gezeigt ist. Dieses Auflageteil weist zwei Auflageflächen 10, 11 auf, die zueinander abgeschrägt sind bzw. aufeinander zulaufen. Dabei ist es vorgesehen, dass der Daumen auf der vorderen Auflagefläche 11 liegt, während der Mittelfinger auf der hinteren Auflagefläche 10 anliegt und der Zeigefinger auf der abgerundeten Oberseite des Auflageteils 4. Diese Finger können den Highspeedmotor völlig entspannt und ohne Ermüdung lagestabilisieren und führen. Hierbei kommt der Schwerpunkt des Motors in der Handsenke zwischen Daumen und Zeigefinger zu liegen und der Daumen liegt zum Handballen hin auf der speziell dafür ausgebildeten und symmetrisch konstruierten Ablagefläche des Auflageteils auf. Das Auflageteil befindet sich im vorderen Gehäusebereich in der Nähe der Abwinkelungsstelle.

Das Auflageteil 4 ist ferner mit einem Verriegelungsmechanismus für ein ebenfalls im vorderen Gehäuse gebildetes Aufnahmefutter 7 für einen Fräserstift 8 verbunden. Das Aufnahmefutter ist damit lösbar verriegelbar und die eingesteckten Fräserstifte 8 lassen sich bequem ver- und entriegeln. Zu diesem Zweck ist das Auflageteil 4 längsverschieblich gelagert und mit dem Verriegelungsmechanismus wirksam verbunden. Durch Verschieben des Auflageteils lassen sich daher im Betrieb die Fräserstifte wechseln.

Zur Führung der Fräserstifte und zu deren Abdeckung ist ein Schaftaufsatz 2 mit sich daran anschließender Hüllmutter 3 vorgesehen, wie es in Fig. 1 deutlich dargestellt ist. Die Hüllmutter 3 ist an ihrer Oberfläche ebenso wie die Ablageflächen 10, 11 profiliert, um eine sichere Handhabung zu ermöglichen. Die Hüllmutter 3 wird auf dem vorderen bzw. stirnseitigen Ende des Gehäuseteils 1a aufgeschraubt und damit fixiert. Im verriegelten Zustand des Fräserstiftes 8 liegt das Auflageteil 4 spaltlos an der Hüllmutter 3 an, während in Fig. 1, 2 das Auflageteil in seiner nach rechts verschobenen Entriegelungsposition für den Fräserstift 8 gezeigt ist. Somit läßt sich der ordnungsgemäße Verriegelungszustand des Fräsers 8 auch optisch beurteilen.

Das Auflageteil hat im Querschnitt etwa die Form eines stilisierten gleichschenkligen Dreiecks, wie man aus Fig. 4 entnehmen kann. Es ist auf dem vorderen Gehäuseteil verdrehsicher und längsverschieblich gelagert und in der Verriegelungsposition durch eine Feder vorgespannt.

Die Hüllmutter 3 des Schaftaufsatzes 2 ist auch als Handhabe für die Fingerspitzen ausgebildet und dazu mit Längsrillen versehen, die auch in den Ablageflächen 10, 11 des Auflageteils 4 vorgesehen sind.

Der Durchmesser des vorderen Motorgehäuseteils 1a ist kleiner als 15 mm, um ein übersichtliches Arbeiten zu ermöglichen. Sämtliche Schaftführungen sind somit wesentlich leichter und verbilligt und die Handhabung wird erleichtert, da kein zusätzlicher Arretiermechanismus und auch kein zusätzliches Winkelgetriebe benötigt wird.

Für enge und tiefe Operationszugänge ist es von Vorteil, den Highspeedmotor auch im hinteren Bereich mit einem Winkeladapter abzuwinkeln. Damit kann der Highspeedmotor auch bei tiefen Eingriffen mit demselben ergonomischen Vorteil weiter hinten gehalten werden. Der zusätzliche Adapter schafft eine Abwinkelung und ist mit dem luftführenden Schlauch bzw. elektrischen Verbindungskabel verbunden.

## Patentansprüche

1. Highspeedmotor für die chirurgische Knochenbearbeitung und Mikrotrepanation, mit einem Motorgehäuse (1, 1a) und einem Aufnahmefutter (7) für einen Fräserstift (8), wobei das Aufnahmefutter (7) einen lösbaren Verriegelungsmechanismus (9) aufweist, dadurch gekennzeichnet, dass das Motorgehäuse (1, 1a) einstückig und abgewinkelt ausgeführt ist und in seinem vorderen Bereich (1a) ein Auflageteil (4) für die Fingerablage des Benutzers aufweist.

2. Highspeedmotor nach Anspruch 1, dadurch gekennzeichnet, dass das Auflageteil zwei einander gegenüberliegende Auflageflächen (10, 11) aufweist.

3. Highspeedmotor nach Anspruch 1 oder 2, dadurch gekennzeichnet, dass die Auflageflächen (10, 11) gegeneinander abgeschrägt sind.

4. Highspeedmotor nach einem der vorhergehenden Ansprüche, dadurch gekennzeichnet, dass das Auflageteil (4) verschieblich gelagert und mit dem Verriegelungsmechanismus (9) verbunden ist.

5. Highspeedmotor nach einem der vorhergehenden Ansprüche, dadurch gekennzeichnet, dass ein Kupplungsstück (12) mit dem hinteren Bereich (1) des Motorgehäuses verbunden ist, wobei das Kupplungsstück (12) für den Anschluß einer Antriebsmittel-Zuleitung (5) dient.

6. Highspeedmotor nach einem der vorhergehenden Ansprüche, dadurch gekennzeichnet, dass zur Führung und Abdeckung des Fräserstiftes (8) ein Schaftaufsatz (2) vorgesehen ist, der eine Hüllmutter (3) aufweist, wobei die Hüllmutter am Motorgehäuse (1a) befestigbar ist.

7. Highspeedmotor nach einem der vorhergehenden Ansprüche, dadurch gekennzeichnet, dass die Winkelung des Gehäuses 20° bis 45° beträgt.

8. Highspeedmotor nach einem der vorhergehenden Ansprüche, dadurch gekennzeichnet, dass der vordere Bereich (1a) gegenüber dem hinteren Bereich (1) des Motorgehäuses im Querschnitt verkleinert ist.

9. Highspeedmotor nach einem der vorhergehenden Ansprüche, dadurch gekennzeichnet, dass das Auflageteil (4) ohne wesentlichen Spalt an der Hüllmutter (3) anliegt, wenn der Fräserstift (8) verriegelt ist.
